# EUROPÄISCHE PATENTSCHRIFT

(11) **EP 1 904 012 B1**
(45) Veröffentlichungstag und Bekanntmachung des Hinweises auf die Patenterteilung: **14.01.2009**
(21) Anmeldenummer: 06754696.0
(22) Anmeldetag: 08.07.2006
(51) Int. Cl.: A61F 13/00

(54) **IN KETTRICHTUNG LÄNGSELASTISCHE BINDE**
BANDAGE WITH LENGTHWISE ELASTICITY IN WARP DIRECTION
BANDAGE A ELASTICITE LONGITUDINALE SENS CHAINE

(30) Priorität: 15.07.2005 DE 102005033720
(43) Veröffentlichungstag der Anmeldung: 02.04.2008
(73) Patentinhaber: Karl Otto Braun GmbH & Co. KG., 67752 Wolfstein (DE)
(72) Erfinder: JUNG, Harald, 67757 Kreimbach-Kaulbach (DE); HÄNSCH, Frauke, 76167 Karlsruhe (DE); SZOMBACH, Karlheinz, 67742 Lauterecken (DE)
(74) Vertreter: Langöhrig, Angelika Beate
(86) Internationale Anmeldenummer: PCT/EP2006/006714
(87) Internationale Veröffentlichungsnummer: WO 2007/009625

(56) Entgegenhaltungen:
- AT-B- 154 588
- DE-A1- 2 240 945
- DE-B- 1 164 021
- US-A- 1 035 107

## Beschreibung

Die Erfindung betrifft eine in Kettrichtung elastische Binde, mit Schussfäden, Dreherfäden sowie elastisch dehnbaren. Steherfäden, wobei die Binde in Dreherbindung gewebt ist, d. h. die Steher- und Schussfäden durch Dreherfäden miteinander verwoben sind. Dabei handelt es sich um ein Drehergewebe vom "Typ Halbdreher". Zu diesem Typus der Halbdreher gehören Gittergewebe, die nur einen Steher und einen Dreher pro Gruppe aufweisen. Drehergewebe weisen insbesondere den Vorteil auf, eine hohe Schiebefestigkeit zu besitzen. Die Schiebefestigkeit eines Gewebes wird durch die Reibungskräfte zwischen den einzelnen Fäden bestimmt.

So ist es beispielsweise aus der DE 100 33 210 A1 bekannt, zur Herstellung eines Stützverbandes einen mit einem härtbaren Reaktionsharz (feuchtigkeitshärtender Kunststoff) beschichteten und/oder imprägnierten textilen Träger zu verwenden, bei dem das Trägermaterial mittels Dreherwebverfahren hergestellt sein kann. Derartige Materialien werden zur Herstellung von sogenannten "Castverbänden" eingesetzt, wobei das Kunststoffmaterial dann vollständig zur Bildung eines rigiden oder semi-rigiden Stützverbandes aushärtet. Eine dauerhafte Elastizität eines derartigen Bindenmaterials ist in diesem Fall nicht erforderlich.

Darüber hinaus ist beispielsweise aus der US-PS 4,207,885 eine elastische Kompressionsbandage bekannt, bei der einzelne Kettfäden in Dreherbindung binden können, um eine Verbesserung der Schiebefestigkeit des Gewebes zu erreichen.

Die Bandage besteht dabei aus einem elastischen Nylongewebe. Derartige Kunstfasern sind jedoch hinsichtlich der Trageeigenschaften, wenn sie auch hinsichtlich der elastischen Eigenschaften gut zu bewerten sind, für die Patienten nicht ausreichend angenehm. So wird es unter dem Gesichtspunkt des Komforts des Patienten insbesondere bevorzugt, wenn entsprechende Bandagen aus natürlichen Fasern hergestellt sind. Darüber hinaus erzeugen solche Binden vergleichsweise hohe Rückstellkräfte bereits bei geringer Dehnung (Ruhedruck).

Zwar sind im Stand der Technik bereits eine Vielzahl von Binden und Bandagen bekannt, dennoch besteht nach wie vor ein Bedarf an elastischen Binden, insbesondere Kurzzugbinden, die einen hohen Arbeitsdruck bei geringem Ruhedruck gewährleisten und zugleich für den Patienten komfortabel zu tragen sind. Darüber hinaus besteht ein Bedarf an Binden, die einer hohen Qualitätsanforderung hinsichtlich ihres dauerhaften Gebrauchs genügen und über die gesamte Zeit der Anwendung ein hohes Maß an Therapiesicherheit bietet.

Die Erfindung löst diese Aufgabe durch eine in Kettrichtung längselastische Binde umfassend ein in Dreherbindung gewebte Textilfläche, mit Schussfäden, Dreherfäden sowie elastisch dehnbaren Steherfäden, bei dem die Steherfäden baumwollelastische Fäden sind. Derartige Textilflächen, insbesondere Gewebe besitzen zum einen eine in weiten Bereichen frei einstellbare Dehnbarkeit sowie insbesondere eine Kompressionskraft in Ruhe des Patienten bei angelegter Binde von < 800 cN/cm Bindenbreite. Auf diese Weise wird ein sehr geringer Ruhedruck erzielt. Ruhedruck ist der ständige Druck, den die Binde in Ruhe, also bei erschlaffter Muskulatur auf das Gewebe und die Gefäße, d. h. auf die zu behandelnde Körperstelle ausübt. Die Kraft, die aufgewandt wird, um die Binde zu dehnen, entspricht demnach der Kraft, die als Ruhedruck auf das Gewebe, d. h. auf die zu behandelnde Körperstelle wirkt. Arbeitsdruck dagegen ist der Druck, den der sich kontrahierende, arbeitende Muskel durch Umfangszunahme dem Widerstand der Kompressionsbandage entgegensetzt.

Aufgrund der vorgesehenen Dreherbindung wird darüber hinaus eine hohe Schiebefestigkeit des Gewebes erreicht und die Binden können ohne weitere Kantenfixierung in jeglicher Breite geschnitten und gewickelt werden. Darüber hinaus wird durch die Verwendung eines Drehergewebes neben der Schiebefestigkeit auch eine gleichmäßige Oberfläche erzielt. Insoweit ist die Herstellbarkeit vereinfacht und die Anmutung verbessert.

Die Verwendung von baumwollelastischen Fäden für das Steherfadensystem besitzt weiterhin den Vorteil, dass die Trageeigenschaften der Binde verbessert werden.

Die Kompressionseigenschaften, insbesondere der im medizinischen Sinne niedrige Ruhedruck und der hohe Arbeitsdruck im Vergleich zu herkömmlichen Kompressionsbinden auf Basis elastischer Polymere, wie Gummi, Polyurethanelastomere, texturierte Polyamid- bzw. Polyestergarne, die einen hohen Ruhedruck bei einem geringen Arbeitsdruck erzeugen, können deutlich verbessert werden. Aber auch gegenüber herkömmlichen Kurzzugbinden auf Basis von Baumwollfäden sind die Kraftdehnungseigenschaften verbessert in Richtung eines weiter gesteigerten niedrigen Ruhedrucks, der annähernd über die gesamte Dehnbarkeit der Binde bestehen bleibt. D. h. die Binde kann annähernd über ihre gesamte Dehnbarkeit mit geringstem Kraftaufwand gedehnt werden. Die Dehnbarkeit kann dabei 30% bis 130% betragen (gemessen nach DIN 61 632). Ein Anstieg auf 10 N/cm Bindenbreite erfolgt erst bei einer relativ hohen Anlegedehnung (Endbereich des Kraftdehnungs-Diagrammes). Unter Anlegedehnung ist hier die Dehnung zu verstehen, mit der eine Binde an eine zu therapierende Gliedmasse angelegt wird, d.h. die Binde wird durch den Anwender auf eine Dehnung von z.B. 50 % gebracht und in dieser Form am Patienten angelegt. Gewünscht ist dabei ein möglichst flacher Kurvenverlauf im Kraft-Dehnungsdiagramm im Bereich von mindestens 2/3 der Gesamtdehnung. Erst nach dem Erreichen des 2. Drittels der Kurve (Dehnbarkeit) ist ein deutlicher Anstieg der Kraft erwünscht. Hierdurch kann eine Binde mit äußerst hoher Therapiesicherheit zur Verfügung gestellt werden, da über einen weiten Bereich der Anlegedehnung ein nahezu konstanter Arbeitsdruck eingestellt werden kann.

Demzufolge weist eine erfindungsgemäße Binde bei einer Dehnung von kleiner 50 % insbesondere kleiner 60 % und ganz besonders bevorzugt kleiner 65 % (bezogen auf die Gesamtdehnung der Binde nach DIN 61 632) eine Kompressionskraft in Ruhe des Patienten bei angelegter Binde von kleiner 800 cN/cm Bindenbreite auf. Damit weist eine erfindungsgemäße Binde eine Rückstellkraft größer 8 N/cm Bindenbreite erst im Bereich von 80-100% der Gesamtdehnbarkeit in Längsrichtung auf.

Zur Einstellung der Elastizität kann entweder vorgesehen sein, dass die Dreherfäden im Wesentlichen unelastisch gestaltet sind, wobei hierbei natürlichen Pflanzenfasern, Fasern tierischen Ursprungs oder Synthesefasern eingesetzt werden. Insbesondere können diese Fäden aus Baumwolle, Zellwolle, Flachs, Wolle, Viskose, Seide und/oder Synthesefasern bestehen. Als Synthesefasern können hierbei bevorzugt Fasern aus Polyamid, Polyester, Polypropylen oder Polyacryl u.a. eingesetzt werden. Alternativ können jedoch auch elastische Dreherfäden eingesetzt werden, beispielsweise ebenfalls baumwollelastische Dreherfäden, so dass sich dann neben einer längselastischen Binde auch eine Elastizität in einer weiteren Richtung ergibt und eine bielastische Binde erzeugt wird.

Weiterhin kann vorgesehen sein, dass die Schussfäden im wesentlichen unelastisch ausgestaltet sind. D. h., wenn eine erfindungsgemäße Binde baumwollelastische Steherfäden und im wesentlichen unelastische Schussfäden aufweist, dann kann je nach Auswahl der Dreherfäden eine bi- oder unelastische Binde erzeugt werden. Alternativ können jedoch auch elastische Schussfäden eingesetzt werden, beispielsweise ebenfalls baumwollelastische Schussfäden, so dass sich dann neben einer längselastischen Binde auch eine Elastizität in einer weiteren Richtung ergibt und eine bielastische Binde erzeugt wird.

Gemäß einer bevorzugten Ausführung der Erfindung kann eine Binde im wesentlichen unelastische Schussfäden, elastische Dreherfäden und baumwollelastische Steherfäden aufweisen. Auf diese Weise kann eine Binde realisiert werden die längs- und querelastisch ist und eine hohe Querstabilität aufweist.

Zur Herstellung von baumwollelastischen Fäden werden entweder Zwirn- oder Spinnkreppfäden verwendet, die in S- und/oder Z-Drehungsrichtung mit Kreppdrehungen von ca. 1100 bis 2400 Touren pro Meter insbesondere mit mehr als 2000 Touren/m in Mit- oder Gegenspinndrahtrichtung versehen sind. Das Bindengewebe wird später einem Krumpfprozess unterzogen, wodurch die Baumwollelastizität ausgelöst wird. Hierbei können alle herkömmlichen Maschinen, die einen Krumpfprozess anwenden zum Einsatz gebracht werden, wie zum Beispiel die Krumpfmaschine shrinkomat sp (Fa. m-tec Maschinenbaugesellschaft mbH, Viersen (Deutschland)).

Die Dreherfäden können dabei die gleiche Dicke aufweisen oder feiner als die Schuss- und/oder Steherfäden sein und insbesondere eine Garnfeinheit von 20 bis 200 dtex aufweisen. Zur Bestimmung der Feinheit soll im Zusammenhang mit der vorliegenden Erfindung das internationale Tex-Einheitensystem verwendet werden, d. h. ein Faden oder Garn ist umso feiner je leichter das Garn in Gramm je 10000 m Lauflänge ist. (dtex)

Zur Einstellung der Dehnbarkeit sind gemäß der vorliegenden Erfindung verschiedene Möglichkeiten gegeben. Einerseits kann die Dehnbarkeit der Binde über den Verkreuzungswinkel α zwischen einem Dreherfaden und einem Steherfaden eingestellt werden. Insbesondere kann dabei der Verkreuzungswinkel α einen Wert von 20-90° einnehmen, so dass eine Dehnbarkeit von 30-130 % resultiert. Darüber hinaus lässt sich die Dehnbarkeit und Kompressionskraft im Fertiggewebe auch über die Kettenfadenspannungen (Dreher- und Steherfaden) bzw. über das Verhältnis der Dreherfadenspannung zu der Steherfadenspannung steuern. Insbesondere weist eine erfindungsgemäße Binde ein Verhältnis von Dreher- zu Steherfadenspannung von 1:5 bis 1:1,5 und eine Dehnbarkeit von 30-130 % auf.

Weitere Möglichkeiten zur Erhöhung bzw. Reduzierung der Dehnbarkeit sind über die Schussdichte gegeben. Es kann erfindungsgemäß vorgesehen sein, dass die Steherfadendichte bei derartigen Binden bei ca. 3 bis 12 Fäden pro Zentimeter liegt. Die Anzahl der Dreherfäden entspricht der Anzahl der Steherfäden pro Zentimeter. Ein derartiges Gewebe besitzt den Vorteil, bis zu 30% leichter als ein entsprechendes Gewebe in Leinwandbindung zu sein.

Die Binde kann insbesondere eine Kompressionsbinde sein, wie sie vorzugsweise im Bereich der Behandlung von venösen Leiden eingesetzt wird. Derartige Binden sind vorzugsweise Kurzzugbinden, um ein Abschnüren der Blutzirkulation in der Gliedmaße, an der sie angelegt wird, zu vermeiden.

Dabei vollzieht eine derartige Binde eine elastische reversible Formveränderung beim Anlegen und besitzt vorzugsweise eine Rückstellung von > 50%, so dass ein mehrfaches verwenden ohne Verlust der Kompressionswirkung möglich ist.

Je nach Auswahl der elastischen Fäden weist die Binde eine Längselastizität sowie gegebenenfalls eine Querelastizität auf, bei gleichzeitig guter Querstabilität.

Im Folgenden sind zwei Beispiele für entsprechende Gewebe angegeben, wie sie bevorzugt zur Realisierung einer Kompressionsbinde gemäß der Erfindung eingesetzt werden können.

### Beispiel 1:

| | |
|---|---|
| Steherfaden: | 20 tex x 2 Baumwolle T/m 2150 zz/S |
| | 20 tex x 2 Baumwolle T/m 2150 ss/Z |
| Kettfolge: | 2 S - 2 Z |
| Kettfadenzahl/10 cm: | 100 |
| Dreherfaden | 10 tex x 2 Zellwolle T/m 600 zz/S |
| Dreherfadenzahl: | 100/10 cm |
| | |
| Steherfaden/Dreherfadenspannung: | 4:1 |
| | |
| Kettfolge: | glatt |
| | |
| Schussmaterial: | 73 tex x 1 Baumwolle |
| Schussdichte: | 78/10 cm gedehnt |
| qm-Gewicht gedehnt: | 112 g |
| | (Dehnung bei 10 N/cm Bindenbreite) |
| | |
| Dehnbarkeit: | 60% |
| Elastischer Wirkungsgrad: | 20% |
| | |
| Kompressionskraft | |
| bei 50% Dehnung: | 3 N/cm Bindenbreite |
| bei 55% Dehnung: | 7 N/cm Bindenbreite |
| bei 60% Dehnung | 8 N/cm Bindenbreite |

### Beispiel 2:

| | |
|---|---|
| Steherfaden: | 20 tex x 2 Baumwolle T/m 2150 zz/S |
| | 20 tex x 2 Baumwolle T/m 2150 ss/Z |
| Kettfolge: | 2 S - 2 Z |
| Kettfadenzahl/10 cm: | 100 |
| | |
| Dreherfaden: | PES-Glattgarn 44 dtex x 1 |
| Dreherfadenzahl/10 cm: | 100 |
| | |
| Steherfaden-/Dreherfadenspannung: | 2:1 |
| | |
| Kettfolge: | glatt |
| | |
| Schussmaterial: | 50 tex x 1 Baumwolle |
| Schussdichte: | 70/10 cm gedehnt |
| qm-Gewicht gedehnt: | 93 g |
| | (Dehnung bei 10 N/cm Bindenbreite) |
| | |
| Dehnbarkeit: | 70% |
| Elastischer Wirkungsgrad: | 18% |
| | |
| Kompressionskraft | |
| bei 50% Dehnung: | 2 N/cm Bindenbreite |
| bei 60% Dehnung: | 3 N/cm Bindenbreite |
| bei 70% Dehnung: | 8 N/cm Bindenbreite |

Bei den hier gezeigten zwei Beispielen handelt es sich um Drehergewebe, bei denen die Dreherfäden aus unelastischen Fäden bestehen und durch die Dreherfäden die Verbindung der Steher- mit den Schussfäden erreicht wird.

Dabei wurde die Kompressionskraft jeweils wie folgt bestimmt. Die Messung der Dehnbarkeit erfolgte dabei nach folgender Methode DIN 61632.

Die durch die Gewebe erhaltenen Binden werden in einer Breite über 200 cm hergestellt und anschließend einem Krumpfprozess unterzogen. Anschließend wird das Breitgewebe in Binden geschnitten, ohne dass eine Kantenfixierung zusätzlich durchzuführen ist. Die dadurch erhaltenen Bänder werden abgelängt und zu Binden gewickelt.

Durch Verwendung verschiedener Kombinationen von Dreherfaden zu Steherfaden, also der Kettfäden zueinander, sowie der Vorsehung verschiedener Spannungen auf dem Dreher- bzw. dem Steherfaden sowie der Verkreuzungswinkel, können bevorzugte Materialparameter der Binde, wie das Kraft-Dehnungsverhalten und insbesondere die Gesamtdehnbarkeit etc. eingestellt werden.

Die Erfindung soll im Folgenden weiter anhand einer Zeichnung erläutert werden, dabei zeigt:
- Figur 1: eine Darstellung von Drehergeweben, wie sie für die längselastische Binde gemäß der Erfindung eingesetzt werden und
- Figur 2: Kraftdehnungsdiagramme.

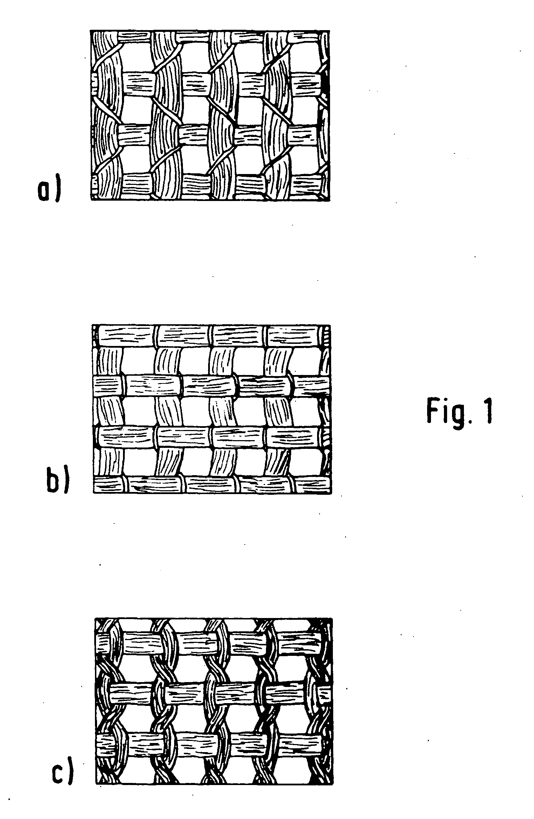

Die Beispiele für Drehergewebe sind in Figur 1 dargestellt, wobei Darstellung a) ein Gewebe mit verschiedenen Kettgarnen, also Dreher- und Steherfäden, und verschiedene Spannungen von der Unterseite betrachtet zeigt. Darstellung b) zeigt die Oberseite des Gewebes aus Darstellung a). Hingegen zeigt Darstellung c) einen Dreherfaden mit gleich starken Kettfäden, aber unterschiedlichen Elastizitäten von Dreher-und Steherfäden. Deutlich zu erkennen ist die Beeinflussung des Gewebes durch die verschiedenen Parameter.

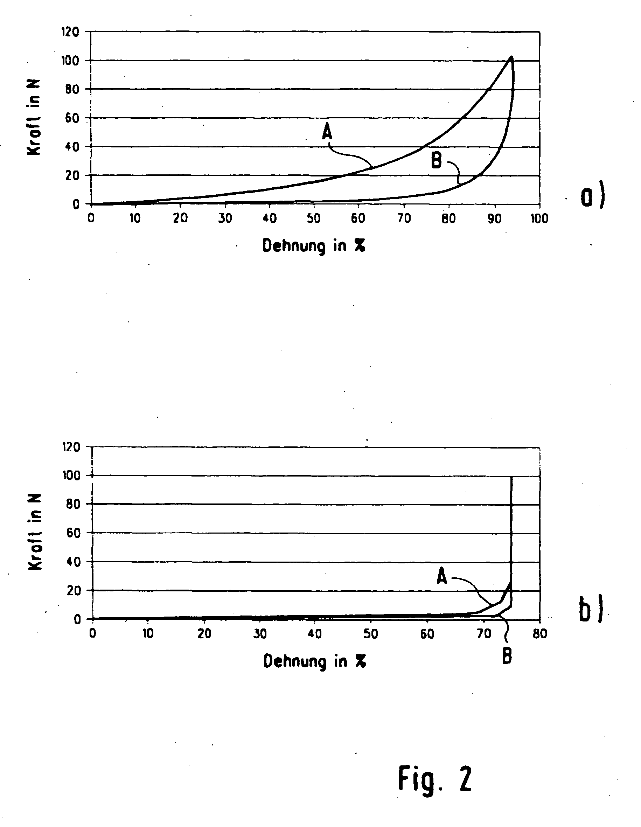

In Figur 2 sind Kraftdehnungsdiagramme dargestellt, wobei das Diagramm a) das Kraftdehnungsverhalten einer bäumwollelastischen Kompressionsbinde nach dem Stand der Technik zeigt.

Figur 2b) zeigt dahingegen eine Kompressionsbinde gemäß der Erfindung hinsichtlich ihrer Kraftdehnungseigenschaften. Die Binde entspricht dabei der Binde aus Beispiel 1. Die Binde besitzt eine Breite von 50 mm.

Bei einer Kurzzugbinde, wie sie in Darstellung a) gezeigt ist, ist die mit A bezeichnete Kurve die Belastungskurve, d. h. die Kurve, die beim Dehnen der Binde beim Anlegen an einen Patienten erreicht wird. Dabei kann ersehen werden, dass bereits frühzeitig, d. h. bei schon bei geringer Dehnung eine erste Kraft durch die Binde aufgebracht wird, wobei diese Kraft dem Ruhedruck äquivalent ist. Die Kurve steigt dann im Bereich einer Dehnung von 90% stark an. Insgesamt besteht jedoch bereits bei einer Dehnung von > 50% eine signifikante, nicht zu vernachlässigende Kraft. Die mit B bezeichnete Kurve symbolisiert die Entlastungskurve, wodurch ersehen werden kann, dass eine derartige baumwollelastische Kurzzugbinde ein deutliches Hystereseverhalten aufweist. Dahingegen ist in Darstellung b) eine erfindungsgemäße Kurzzugbinde gezeigt. Die Figur zeigt, dass bis zu einer Dehnung von ca. 75% annähernd keine Kraft aufgewendet werden muss. D. h., die Binde lässt sich annähernd ohne Kraft ziehen, bis dann bei ca. 75% der Dehnung die Kraftkurve steil nach oben steigt, also die Dehnung begrenzt ist und eine weitere Dehnung nicht mehr bzw. nur noch mit einem sehr viel höherem Kraftaufwand möglich ist. Auch hier ist die Belastungskurve mit A und die Entlastungskurve mit B bezeichnet, wobei hier ein deutlich verringertes Hystereseverhalten zu erkennen ist. Auf diese Weise lässt sich gut erkennen, dass eine derartige erfindungsgemäße Bandage nur einen äußerst geringen, annähernd vernachlässigbaren Ruhedruck auf eine Gliedmaße ausübt, an der die entsprechende Binde angelegt ist. Darüber hinaus wird bei Anwickeln der Binde bis in den Bereich kurz vor der Maximaldehnung und Anstieg der Kraft erreicht, dass bei Bewegung der Gliedmaße oder des Körperteils die Kraft auf die Gliedmaße verhältnismäßig groß ist und so ein günstiger Arbeitsdruck erreicht wird, wobei der Arbeitsdruck mit dem Muskeltonus und der Bewegung des Körperteils korrespondiert.

Insgesamt lässt sich auf die vorstehende Weise eine Binde herstellen, die gegenüber herkömmlichen Binden den Vorteil besitzt, dass Binden jeglicher Breite ohne vorgefertigte Gassen und zusätzliche Kantenfixierung aus einem Breitgewebe geschnitten werden können und durch die Auswahl der Kettfadenspannung, der Fadenmaterialien und des Kreuzungswinkels zwischen Dreher- und Steherfaden die Dehnbarkeit und Kompressionskraft des Fertiggewebes gesteuert werden kann. Darüber hinaus ist ein entsprechendes Gewebe bis zu 30% leichter bei verbesserter Schiebefestigkeit.

## Patentansprüche

1. In Kettrichtung längselastische Binde umfassend eine in Dreherbindung gewebte Textilfläche, mit Schussfäden, Dreherfäden sowie elastisch dehnbaren Steherfäden, bei der die Steherfäden baumwollelastische Fäden sind, **dadurch gekennzeichnet, dass** die Dreherfäden im Wesentlichen unelastisch sind.

2. Binde nach Anspruch 1, **dadurch gekennzeichnet, dass** die Schussfäden im wesentlichen unelastisch sind.

3. Binde nach einem der vorangehenden Ansprüche, **dadurch gekennzeichnet, dass** die Dreherfäden aus Baumwolle, Flachs, Wolle, Seide, Viskose, Zellwolle und/oder Synthesefasern bestehen.

4. Binde nach einem der vorangehenden Ansprüche, **dadurch gekennzeichnet, dass** die Dreherfäden feiner als die Steherfäden sind.

5. Binde nach einem der vorangehenden Ansprüche, **dadurch gekennzeichnet, dass** die Steherfäden Baumwollkreppfäden, insbesondere Zwirnkreppfäden sind.

6. Binde nach einem der vorangehenden Ansprüche, **dadurch gekennzeichnet, dass** es sich um eine Kompressionsbinde handelt.

7. Binde nach einem der vorangehenden Ansprüche, **dadurch gekennzeichnet, dass** die Binde in Längsrichtung eine Dehnbarkeit von 30-130% aufweist.

8. Binde nach einem der vorangehenden Ansprüche, **dadurch gekennzeichnet, dass** über den Verkreuzungswinkel von Steherfäden und Dreherfäden die Dehnbarkeit und die Dehnungskraft einstellbar ist.

9. Binde nach einem der vorangehenden Ansprüche, **dadurch gekennzeichnet, dass** eine Rückstellkraft der Binde größer 8 N/cm Bindenbreite erst im Bereich von 80-100% der Gesamtdehnbarkeit in Längsrichtung erreicht wird.

10. Binde nach einem der vorangehenden Ansprüche, **dadurch gekennzeichnet, dass** nach dem Weben, die Binde zur Erreichung der Baumwollelastizität einer Krumpfbehandlung unterzogen ist.

## Claims

1. A bandage with lengthwise elasticity in warp direction, comprising a textile surface woven in leno weave, with weft threads, leno threads as well as elastically extensible core threads, wherein the core threads are cotton-elastic threads, **characterized in that** the weft threads are substantially inelastic.

2. Bandage according to claim 1, **characterized in that** the weft threads are substantially inelastic.

3. Bandage according to one of the above claims, **characterized in that** the leno threads are made of cotton, flax, wool, silk, viscose, rayon staple and/or synthetic fibers.

4. Bandage according to one of the above claims, **characterized in that** the leno threads are finer than the core threads.

5. Bandage according to one of the above claims, **characterized in that** the core threads are cotton crepe threads, in particular, ply yarn crepe threads.

6. Bandage according to one of the above claims, **characterized in that** it is a compression bandage.

7. Bandage according to one of the above claims, **characterized in that** the bandage has a lengthwise extensibility of 30-130%.

8. Bandage according to one of the above claims, **characterized in that** the extensibility and elongation force can be set via the crossing angle between core threads and leno threads.

9. Bandage according to one of the above claims, **characterized in that** a restoring force of the bandage larger than 8 N/cm bandage width is just achieved at a range of 80-100% of the total lengthwise extensibility.

10. Bandage according to one of the above claims, **characterized in that**, after weaving, the bandage is subjected to a shrinking process for achieving the cotton elasticity.

## Revendications

1. Bandage à élasticité longitudinale dans le sens de la chaîne comportant une surface textile tissée en armure croisée, avec des fils de trame, des fils de tour ainsi que des fils droits extensibles, dont les fils droits sont des fils en coton élastiques, **caractérisé en ce que** les fils de tour ne sont pas pour l'essentiel élastiques.

2. Bandage selon la revendication 1, **caractérisé en ce que** les fils de trame ne sont pas pour l'essentiel élastiques.

3. Bandage selon l'une quelconque des revendications précédentes **caractérisé en ce que** les fils de tour sont en coton, en lin, en laine, en soie, en viscose, en laine de cellulose et/ou en fibres synthétiques.

4. Bandage selon l'une quelconque des revendications précédentes, **caractérisé en ce que** les fils de tour sont plus fins que les fils droits.

5. Bandage selon l'une quelconque des revendications précédentes, **caractérisé en ce que** les fils droits sont des fils crêpés en coton, en particulier des fils crêpés retordus.

6. Bandage selon l'une quelconque des revendications précédentes, **caractérisé en ce qu'**il s'agit d'un bandage de compression.

7. Bandage selon l'une quelconque des revendications précédentes, **caractérisé en ce qu'**il présente une extensibilité de 30 à 130 % dans le sens longitudinal.

8. Bandage selon l'une quelconque des revendications précédentes, **caractérisé en ce qu'**il est possible d'ajuster l'extensibilité et la force d'allongement avec l'angle de croisement de fils droits et de fils de tour.

9. Bandage selon l'une quelconque des revendications précédentes, **caractérisé en ce qu'**on atteint une force de rappel du bandage supérieure à 8 N/cm de largeur de bande uniquement dans le domaine de 80 à 100 % de l'extensibilité totale dans le sens longitudinal.

10. Bandage selon l'une quelconque des revendications précédentes, **caractérisé en ce qu'**on soumet le bandage, après le tissage, à un traitement anti-rétrécissement afin d'obtenir l'élasticité du coton.
